# EUROPEAN PATENT SPECIFICATION

(11) **EP 2 988 236 B1**
(45) Date of publication and mention of the grant of the patent: **05.06.2019**
(21) Application number: 15181732.7
(22) Date of filing: 20.08.2015
(51) Int. Cl.: G16H 50/70, G06N 5/04, G16H 50/20

(54) **PREDICTIVE MODEL GENERATOR**
PRÄDIKTIVMODELLGENERATOR
GÉNÉRATEUR DE MODÈLE PRÉDICTIF

(30) Priority: 20.08.2014 US 201414464330
(43) Date of publication of application: 24.02.2016
(73) Proprietor: Accenture Global Services Limited, Dublin 4 (IE)
(72) Inventor: Carroll, Dennis, Leander, TX Texas 78641 (US); Lynch, Cecil, Granite Bay, CA California 95746 (US); Acuna, German, Austin, TX Texas 78756 (US); Vo, Anh-Hoang, Austin, TX Texas 78732 (US)
(74) Representative: Conroy, John

(56) References cited:
- US-A1- 2011 295 622
- US-A1- 2013 262 357
- US-B1- 6 269 351
- HONGMEI SHAO ET AL: "A New BP Algorithm with Adaptive Momentum for FNNs Training", INTELLIGENT SYSTEMS, 2009. GCIS '09. WRI GLOBAL CONGRESS ON, IEEE, PISCATAWAY, NJ, USA, 19 May 2009 (2009-05-19), pages 16-20, XP031516219, ISBN: 978-0-7695-3571-5

## Description

### BACKGROUND

In some industries, such as the healthcare industry, predicted outcomes may be used to formulate various services that are offered to customers. Using the healthcare industry as an example, medical characteristics of a patient may be analyzed to predict the likelihood of a future hospitalization based on hospitalization data that is available for patients that have been previously hospitalized. In some examples, patient service providers (e.g., medical practitioners) and payers (e.g., insurance providers) may be interested in predicting a hospitalization risk of the patient in an efficient manner. In other examples, predicted outcomes may be used to affect business practices in other, non-healthcare related industries.

HONGMEI SHAO ET AL: "A New BP Algorithm with Adaptive Momentum for FNNs Training" discloses a backpropagation algorithm with adaptive momentum, where the momentum coefficient is adjusted iterativelv based on the current descent direction and the weight increment in the last iteration.

US 6 269 351 B1 discloses a method and system for training an artificial neural network.

US 2013/262357 A1 discloses a clinical predictive and monitoring system and method.

US 2011/295622 A1 discloses a healthcare information technology system for predicting or preventing readmissions.

### SUMMARY

The present invention is defined bv the independent claims.

Particular embodiments are defined by the subject-matter of the dependent claims.

Embodiments which do not fall within the scope of the claims are to be interpreted merely as examples useful for understanding the invention.

In accordance with implementations of the present disclosure, techniques employed to analyze data can be carried out relatively quickly to provide a versatile predictive model, as compared to conventional techniques that may be employed to generate predictive models. Furthermore, implementations of the present disclosure can be used to quickly generate any of several forms of predictive models that are based on various populations and that can predict any one of several different output values. That is, implementations of the present disclosure are flexible enough to be used for multiple applications and may be used across a wide variety of industries or multi-disciplinary industries.

The present disclosure further provides a system for implementing the methods provided herein. The system includes one or more processors, and a computer-readable storage medium coupled to the one or more processors having instructions stored thereon which, when executed by the one or more processors, cause the one or more processors to perform operations in accordance with implementations of the methods provided herein.

It is appreciated that methods in accordance with the present disclosure can include any combination of the aspects and features described herein. That is, methods in accordance with the present disclosure are not limited to the combinations of aspects and features specifically described herein, but also include any combination of the aspects and features provided.

The details of one or more implementations of the present disclosure are set forth in the accompanying drawings and the description below. Other features and advantages of the present disclosure will be apparent from the description and drawings, and from the claims.

### BRIEF DESCRIPTION OF DRAWINGS

FIG. 1 depicts an example system that can execute implementations of the present disclosure.
FIG. 2 depicts an example computing environment in accordance with implementations of the present disclosure.
FIG. 3A depicts an example algebraic model.
FIG. 3B depicts an example tree-based model.
FIGS. 4A and 4B depict example tables providing example momentum determinations in accordance with implementations of the present disclosure.
FIG. 5 depicts an example process that can be executed in implementations of the present disclosure.
FIG. 6 depicts an example computing system that can execute implementations of the present disclosure.

### DETAILED DESCRIPTION

Implementations of the present disclosure are generally directed to providing a predictive model. In some examples, the predictive model predicts an output value based on one or more input values. In some implementations, the predictive model is provided based on an iterative refinement process. More particularly, a momentum analysis is provided based on modified simulated annealing and momentum multipliers. In some examples, the momentum of a change to a model parameter is used to iteratively adjust the parameter, as described herein. In some examples, one or more computer programs implemented on one or more devices of a computer system may receive data stored on one or more devices of the computer system, and can process the data to provide the predictive model. In some examples, the data is historical data. In some examples, the data is real-time data. The one or more computer programs may analyze the data to produce an initial version of the predictive model and subsequently refine the initial version of the predictive model to provide a final version of the predictive model. In accordance with implementations of the present disclosure, techniques employed to analyze the data can be carried out relatively quickly to provide a versatile predictive model, as compared to conventional techniques that may be employed to generate predictive models. Furthermore, implementations of the present disclosure can be used to quickly generate any of several forms of predictive models that are based on various populations and that can predict any one of several different output values.

Implementations of the present disclosure are described herein in a non-limiting, example context. The example context includes the healthcare industry. Implementations of the present disclosure are described in further detail herein with reference to an example predicted (output) value in view of the example context. The example value includes patient hospitalization risk (e.g., unplanned hospitalization risk). In this example, the hospitalization risk of a particular patient can be predicted based on actual hospitalization data (e.g., existing, historical data) that is available from a population (e.g., a sample or a pool) of patients that have been previously hospitalized or that is provided as real-time data as the data is being collected from one or more patients. It is appreciated, however, that implementations of the present disclosure are applicable in other contexts and/or with other values. In general, the present disclosure is directed to providing a predictive model for predicting a value based on available data. Accordingly, for the example context of the healthcare industry, the other values that may be predicted by the model can include a duration of a hospital stay, a perinatal cost for a newborn based on maternal health history, and a risk of congestive heart failure, among several other predicted values. Implementations of the present disclosure may also be used to predict values related to other, non-healthcare industries, such as other services industries, the automotive industry, the agricultural industry, and any number of other industries. That is, implementations of the present disclosure are flexible enough to be used for multiple applications and may be used across a wide variety of industries or multi-disciplinary industries.

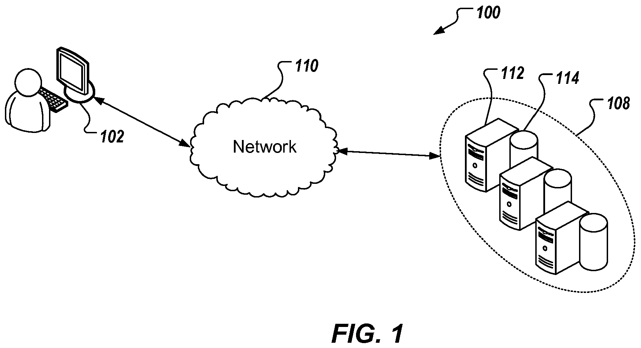

FIG. 1 depicts an example system 100 that can execute implementations of the present disclosure. In the depicted example, the system 100 includes a computing device 102 that communicates with a server system 108 over a network 110. In some examples, the computing device 102 can represent various forms of processing devices including, but not limited to, a desktop computer, a laptop computer, a tablet computer, a handheld computer, a personal digital assistant (PDA), a cellular telephone, a network appliance, a camera, a smart phone, an enhanced general packet radio service (EGPRS) mobile phone, a media player, a navigation device, an email device, a game console, or a combination of any two or more of these data processing devices or other data processing devices. As discussed in further detail herein, the computing device 102 can interact with application software provided in the server system 108.

In some implementations, the server system 108 can include one or more servers 112 and databases 114. In some examples, the servers 112 can represent various forms of servers including, but not limited to a web server, an application server, a proxy server, a network server, or a server farm. For example, the servers 112 can be application servers that execute software accessed by computing devices 102, 104. In operation, multiple computing devices 102, 104 (e.g., clients) can communicate with the servers 112 by way of the network 110. In some implementations, a user can invoke applications available on the servers 112 in a user-interface application (e.g., a web browser) running on the computing device 102. Each application can individually access data from one or more repository resources (e.g., databases 114).

In some implementations, the system 100 can be a distributed client/server system that spans one or more networks such as network 110. The network 110 can be a large computer network, such as a local area network (LAN), wide area network (WAN), the Internet, a cellular network, or a combination thereof connecting any number of mobile clients, fixed clients, and servers. In some implementations, each client (e.g., computing device 102) can communicate with the server system 108 through a virtual private network (VPN), Secure Shell (SSH) tunnel, or other secure network connection. In some implementations, the network 110 can include the Internet, a wireless service network, and may include the Public Switched Telephone Network (PSTN). In other implementations, the network 110 may include a corporate network (e.g., an intranet) and one or more wireless access points.

Within the non-limiting example context discussed above, implementations of the present disclosure will be described for the example prediction of determining a hospitalization risk (e.g., an unplanned hospitalization risk) of a patient. In some examples, the hospitalization risk may be utilized by one or more parties that provide services to the patient in order to make determinations about how to formulate or modify those services. For example, medical practitioners (e.g., doctors, nurses, or therapists) may use the hospitalization risk to generate one or more of a prognosis and a treatment plan for the patient. In some examples, payers, such as insurance providers (e.g., health insurance providers or life insurance providers), may use the hospitalization risk to perform one or more of selecting insurance coverage features (e.g., eligible medical services, eligible medical providers, coverage periods, or coverage amounts), determining insurance premiums, and allocating medical resources (e.g., medical equipment or staffing) within a treatment facility.

In some implementations, the hospitalization risk of a patient may be predicted based on actual hospitalization data that is available from a population of patients that have been previously hospitalized due to a variety conditions or data that is available in real-time data as the data is being collected from one or more patients. More particularly, computer programs implemented on the server system 108 of the system 100 may perform a hybrid algorithm that is based in part on simulated annealing and binary search analyses using the actual hospitalization data to generate a model for predicting the hospitalization risk as a function of one or more features (e.g., biological parameters, medical conditions, other patient characteristics, or other medical history parameters). In this manner, the predictive model may identify which conditions are likely to cause an unplanned hospitalization. Once generated, the predictive model may be used to calculate a predicted hospitalization risk of a particular patient with respect to the one or more features. With reference to a threshold risk, the predictive model may therefore identify patients that are susceptible to an unplanned hospitalization.

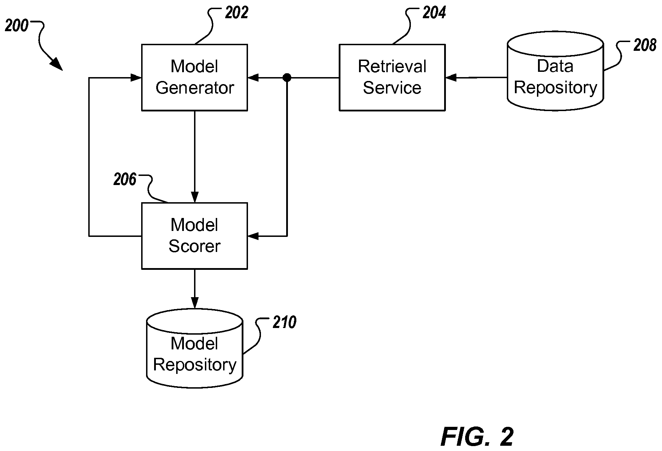

FIG. 2 depicts an example computing environment 200 in accordance with implementations of the present disclosure. In some implementations, the computing environment 200 may include a data repository 208 and a model generator 202 that receives (e.g., indirectly receives) actual (e.g., historical or real-time) data (e.g., hospitalization data for a patient population) from the data repository 208 as input. For example, the computing environment 200 may include a retrieval service 204 that mediates between the model generator 202 and the data repository 208. In some examples, the retrieval service 204 may query the data repository 208, receive the actual data from the data repository 208 in response to the query, and provide the actual data to the model generator 202. The model generator 202 and the retrieval service 204 may be implemented on one or more of the servers 112 of the system 100, and the data repository 208 may provide an implementation of one or more of the databases 114 of the system 100. The data repository 208 may be populated an extract, transform, load (ETL) component that receives data files and loads data from the data files into the data repository. The actual data may include, for each entity (e.g., patient) represented in the actual data, a target value associated with a target parameter (e.g., a value corresponding to a hospitalization risk) and multiple feature values associated with multiple features (e.g., values corresponding to various measured or otherwise quantified medical parameters or other patient characteristics). In some examples, the multiple features, when embodied as medical parameters, may reflect Diagnostic and Statistical Manual of Mental Disorders (DSM) codes, Current Procedural Terminology (CPT) codes, International Classification of Disease (ICD)-9 diagnosis codes, hierarchical condition codes (HCC), Systematized Nomenclature of Medicine (SNOMED) codes, Logical Observation Identifiers Names and Codes (LOINC) laboratory data, RxNORM prescription data, or other types of codes that reflect various morbidities or pathophysiologies. In some examples, the multiple features may include or reflect demographic information, such as age, gender, race, income, or zip code. In some examples, the actual data may be stored in one or more tables, where each row or column corresponds to a particular entity represented in the actual data. In some examples, the actual data may include multiple target values respectively associated with multiple target parameters for each entity represented in the actual data.

In some implementations, the model generator 202 may generate a model for predicting a target value associated with a target parameter represented in the actual data stored in the data repository 208. In some examples, a user may select a type of the predictive model (e.g., an algebraic model, a tree-based model, or any appropriate type of model) or a variation of the predictive model to be generated by the model generator 202. In some implementations, the user may select the type based on one or more analyses (e.g., a regression analysis or another type of analysis) that has been performed on the actual data.

In some examples, the model generator 202 may select an initial weight corresponding to each of the features represented in the predictive model based on the actual data received from the data repository 208. In some examples, the weights may be initialized with a value of zero, one, or another non-zero value.

In some examples, the model generator 202 adjusts the initial predictive model and iteratively adjusts subsequent versions of the predictive model to refine the predictive model. For example, weights associated with the features represented in the predictive model may be adjusted in an attempt to improve the predictive model with respect to the initial (e.g., unadjusted) state or with respect to an adjusted (e.g., intermediate, non-finalized) state. In some implementations, adjustments to the model may be performed by a module (e.g., a model adjuster) that is included in the computing environment 200 but provided separately from the model generator 202. Such a model adjuster may be implemented on one or more of the servers 112 of the system 100.

In some implementations, the computing environment 200 may further include a model scorer 206 that receives an instantiation of the adjusted predictive model and the actual data stored in the data repository 208. In some examples, the model scorer 206 scores the predictive model based on the actual data. The model scorer 206 may be implemented on one or more of the servers 112 of the system 100. In some examples, once one or more weights associated with a particular feature have been adjusted by the model generator 202, the model scorer 206 may then use the feature values included in the actual data to score the predictive model. For example, the model scorer 206 may calculate the predicted target value for each entity represented in the actual data using the feature values respectively associated with each entity in the actual data as inputs to the model adjusted by the model generator 202. In some examples, the model scorer 206 may calculate an effective predicted value as a total (e.g., a sum), an average, or a median of predicted target values calculated for all or a certain number of the entities represented in the actual data. Furthermore, the model scorer 206 may calculate an effective actual value as a total, an average, or a median of actual target values provided for all or a certain number of the entities represented in the actual data. In some implementations, a scoring functionality may alternatively be provided along with each version of the predictive model (e.g., each adjusted model) and be executed to score the associated predictive model. In some examples, the model scorer 206 or such a scoring functionality may include a scoring plugin that can be implemented to score the model.

In some implementations, the model scorer 206 may calculate a model score as the difference between the effective predicted value (e.g., calculated using the feature values included in the actual data as inputs to the predictive model) and the effective actual value (e.g., calculated using the target values included in the actual data). In some implementations, a reduced model score (e.g., as compared to a previously calculated best model score) indicates that the current model adjustments have improved the model, or in other words, that the effective predicted value has approached the effective actual value. In some implementations, an increased model score (e.g., as compared to a previously calculated best model score) indicates that the current model adjustments have not improved the model, or in other words, that the effective predicted value has not further approached the effective actual value with reference to the best model score.

In some implementations, the model scorer 206 can include a modifying function that may, for example, sort or rank predicted values for each entity of a population. Such rankings can then be compared to actual rankings for each entity of the population. In some implementations, a relatively high model score is achieved for closely matching rankings, whereas as a relatively low model score is achieved for rankings that do not closely match.

In some implementations, the model scorer 206 may map an effective predictive value (e.g., according to classification scoring) to one of several predetermined categories of predicted values and then compare the predetermined category to the actual category. In such implementations, a match of the predetermined category with the actual category can indicate that the model has improved, whereas a predetermined category that is different from the actual category can indicate that the model has not improved. In some examples, such predetermined categories can include defined ranges of values. In some examples, such predetermined categories may correspond to "low risk," "medium-risk," or "high-risk" patient categories.

In some implementations, the model score can be compared to a threshold score. In some examples, if the model score exceeds the threshold score, the predictive model is unsatisfactory, and the model generator 202 again adjusts the one or more weights of the predictive model. In some examples, if the model score does not exceed the threshold score, the predictive model is satisfactory. In some examples, if the predictive model is satisfactory and no further adjustments are to be made (e.g., to one or more other weights), the current version of the predictive model is provided as the final version of the predictive model. In some examples, if the predictive model is satisfactory, it can still be determined that one or more other weights are to be adjusted, and the model generator 202 again adjusts the predictive model.

In some implementations, the model generator 202 iteratively adjusts the predictive model based on a momentum analysis. More particularly, and in accordance with implementations of the present disclosure, weights of the predictive model are adjusted using a momentum analysis based on modified simulated annealing and momentum factors. In some examples, the momentum is the amount (e.g., including a magnitude and a sign or direction) by which a particular weight is adjusted for a current iteration. In some examples, and as described herein, a weight is adjusted by an increment during a first iteration, and the weight is adjusted by a multiple of the increment in subsequent iterations. In some examples, the increment reflects a momentum of change to the weight, and is adjusted at each iteration based on an improvement or lack of improvement to the predictive model. In some examples, iterations are repeated until the momentum reaches a minimum value (e.g., zero) or until a maximum number of iterations have been completed. In some examples, imposing such a maximum number of iterations can prevent the model from including a "runaway" characteristic (e.g., a significant overweighting of a single feature).

In some implementations, a momentum value M may be provided as a scalar value. In some examples, the computing environment 200 may employ an approach that adjusts the predictive model in a prescribed manner using momentum values M. In some implementations, the model scorer 206 iteratively scores the predictive model, and the model generator 202 accordingly iteratively adjusts the predictive model with respect to the particular feature until a final adjustment factor (e.g., a final weight) has been determined for the particular feature. In this manner, the computing environment 200 may employ a simulated annealing approach that repeatedly changes the predictive model, tests the performance of the predictive model in response to the change, and selectively changes the predictive model based on the performance. In some implementations, the predictive model is iteratively adjusted for each of the features represented in the predictive model until a final adjustment factor has been determined for each feature. In some implementations, the predictive model may be updated periodically or in real-time based on revised (e.g., updated) or new data provided to the data repository 208.

In some examples, the computing environment 200 may further include a model repository 210 that receives the predictive model (e.g., a finalized version of the model) from the model scorer 206 once a final adjustment factor has been determined for each feature represented in the predictive model. The model repository 210 may provide an implementationof one or more of the databases 114 of the system 100. In some examples, the model repository 210 may be provided as a directory of XML files.

In some implementations, an example computing environment may be implemented as a cloud-based environment. For example, a model adjuster may be provided in a web service application programming interface (API) that receives actual data and predictive models from respective databases and then provides the data to a scoring module that scores a model based on received data.

The predictive model can be provided in various forms. Example forms, described in further detail herein, include an algebraic model and a tree-based model.

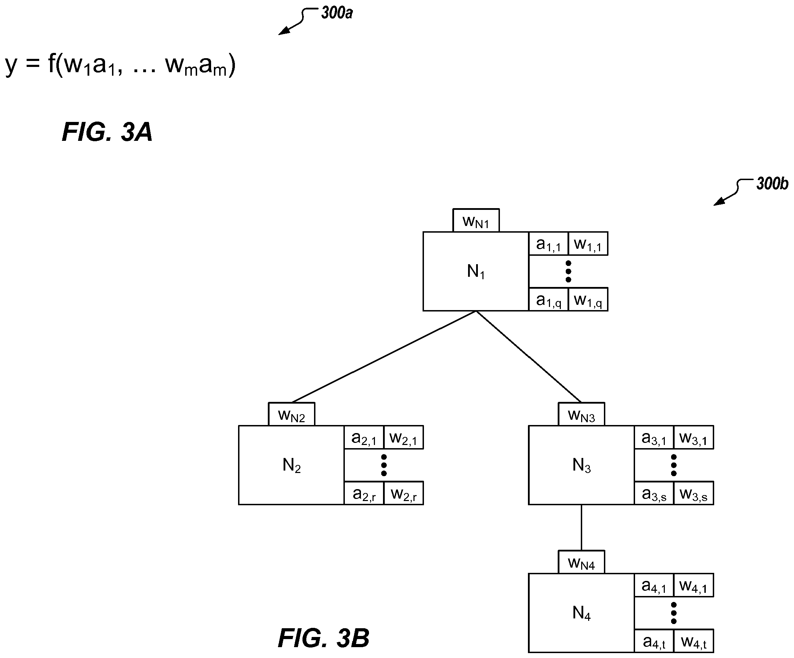

FIG. 3A depicts an example algebraic model 300a that may be generated by the model generator 202. The example algebraic model 300a is an embodiment that does not fall within the scope of the claims. In some implementations, the algebraic model 300a may provide y asa function *f* of multiple variables *a1...am,* where y may correspond to the target parameter represented in the actual data, *a1...am* may correspond to the multiple features represented in the actual data, and *w₁* ... *wₘ* may represent the weights respectively associated with the multiple features. For example, *w1* ... *Wm* may be provided as scalar values. In some implementations, the model 300a may provide a linear function. In some implementations, the model 300a may provide a non-linear function. In some examples, an initial algebraic model can reflect a model that was previously generated based on a different population. In some examples, using such a previously generated model as a starting point for the predictive model may improve accuracy of the predictive model or the speed at which the predictive model converges to a solution. In some examples, an initial algebraic model can be provided based on actual data (e.g., provided from the data repository 208 of FIG. 2). For example, an initial algebraic model can be provided based on a curve-fitting process.

FIG. 3B depicts an example tree-based model 300b that may be generated by the model generator 202. In some implementations, the tree-based model 300b may provide multiple nodes *N₁...Nₘ* that are respectively associated with multipliers *w_{N1}...w_{Nm}* (e.g., *m* = 4 in the example tree-based model 300b). For example, *w_{N1}...w_{Nm}* may be provided as scalar values. In the tree-based model 300b, *N₁* is a root (e.g., top-most) node and is also a parent node of *N₂* and *N₃. N₂* and *N₃* are intermediate nodes and are also child nodes of *N₁. N₃* is a parent node of N₄, and *N₄* is a child node of *N₃. N₂* and *N₄* are also leaf (e.g., terminal) nodes of the tree-based model 300b.

In some examples, the root node *N₁* may correspond to the target parameter represented in the actual data, and the child nodes *N₂, N₃, N₄* may correspond to the multiple features represented in the actual data. Each node *N₁...Nₘ* of the tree-based model 300b accounts for multiple feature values *a_{n,1}*...*a_{n,z}* included in the actual data that are respectively associated with multiple weights *w_{n,1}...w_{n,z}*, where n corresponds to one of the nodes *N₁...Nₘ,* and z is the number of values accounted for by the node (e.g., *q, r, s,* and *t* in the tree-based model 300b). Each node *N₁...Nₘ* of the tree-based model 300b further accounts for any child nodes directly or indirectly associated with the node. For example, *N₁* accounts for *N₂, N₃,* and *N₄,* and *N₃* accounts for *N₄.* The tree-based model 300b may be traversed from a terminal-most leaf node (e.g., *N₄*) up to the root node (e.g., *N₁*) (e.g., according to a depth-first tree traversal) to calculate the predicted target value by multiplying the result of each node *N₁...Nₘ* by the its associated multiplier *w_{N1}...w_{Nm}.* Accordingly, the predicted target value may be calculated by multiplying the result of *N₁* by *w_{N1}.* In some examples, *w_{N1}* may have a value of one. In some examples, each feature *a_{n,1}...a_{n,z}* that exists at a node *N₁...Nₘ* may be multiplied by its associated weight *w_{n,1}...w_{n,z}.* The node *N₁...Nₘ* may then be recursively multiplied by its associated multiplier *w_{N1}...w_{Nm}.* All values defined by the node *N₁...Nₘ* may then be aggregated (e.g., according to a sum or a count), and, in some cases, a condition may be applied to the node. A value for the node *N₁...Nₘ* can then be returned. In some implementations, the model generator 202 may provide a tree-based model that is different from that of the tree-based model 300b. For example, the model generator 202 may provide a tree-based model that provides more than four nodes or less than four nodes.

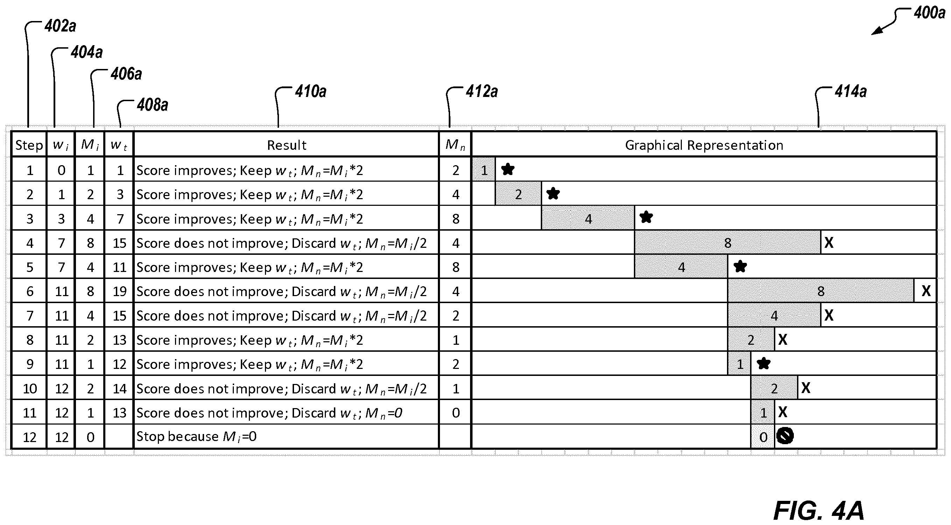

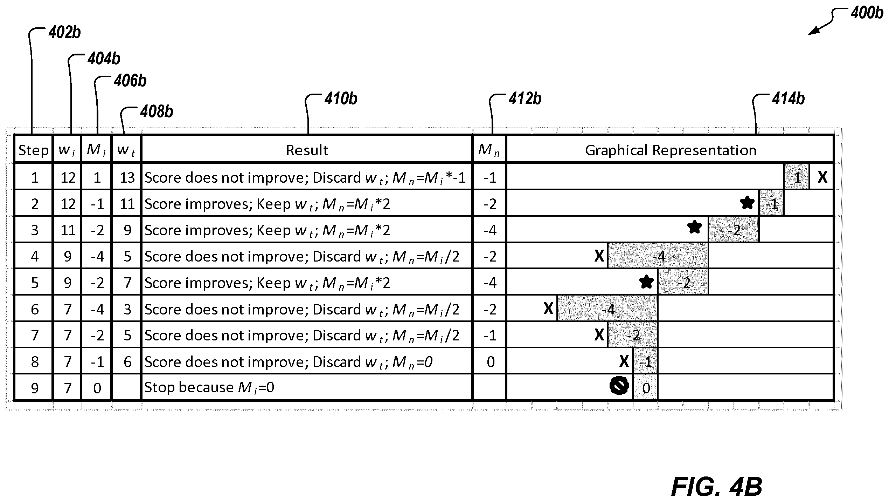

FIGS. 4A and 4B depict example tables 400a, 400b providing example momentum determinations based on simulated annealing and momentum search techniques in accordance with implementations of the present disclosure. Although the example tables of 400a, 400b are processed using multiples of as 2 and ½, it is appreciated that implementations of the present disclosure can be based on using any multiple. For example, other momentum determinations can be based on any combination of multiples, such as 3, 1/3, 4, ¼,5, 1/5, and so on.

With reference to the example tables 400a, 400b, the momentum analysis of the present disclosure will be described in detail. The tables 400a, 400b illustrate the momentum analysis, which combines simulated annealing and momentum search techniques to adjust a predictive model (e.g., the predictive model 300a, 300b) generated by the model generator 202. The tables 400a, 400b include columns 402a, 402b representing a step (iteration) in the process, columns 404a, 404b representing an initial weight *wᵢ* of a feature (e.g., a medical parameter or a patient characteristic) represented in the predictive model for respective iterations, columns 406a, 406b representing an initial momentum *Mᵢ* applied to the initial weight *wᵢ* for respective iterations, columns 408a, 408b representing a test weight *wₜ* of the feature represented in the predictive model for respective iterations, columns 410a, 410b indicating a result of the predictive model implemented with the test weight *wₜ* for respective iterations, columns 412a, 412b representing a next momentum *Mₙ* to be applied in a next iteration of the process for respective iterations, and columns 414a, 414b illustrating a graphical representation of respective iterations in the process.

In some implementations, the model scorer 206 receives the predictive model (e.g., an initial, unadjusted version of the predictive model including initial weights) from the model generator 202 and uses the actual data received from the data repository 208 to score the predictive model prior to execution of the first iteration of the process. In some examples, a weight (e.g., *w₁*...*wₘ* of the model 300a or *w_{N1}...w_{Nm}* or *w_{n,1}...w_{n,z}* of the model 300b) associated with a particular feature or parameter (e.g., *a₁...aₘ* of the model 300a or *a_{n,1}...a_{n,z}* or *N₁...Nₘ* of the model 300b) represented in the predictive model may be iteratively adjusted according to a next momentum *Mₙ* until changing the next momentum *Mₙ* no longer improves the predictive model, and the next momentum *Mₙ* may therefore be set to 0. For example, if changing the predictive model according to *Mₙ* improves the model, then the magnitude of the momentum increases, whereas if changing the predictive model according to *Mₙ* does not improve the model, then the magnitude of the momentum decreases. In some examples, the weight may be adjusted in a positive direction (e.g., the weight may increase) as the process is carried out. In some examples, the weight may be adjusted in a negative direction (e.g., the weight may decrease) as the process is carried out. In some examples, a sign of the weight can change. In some examples, the weight may be adjusted to a final value of zero. In some implementations, the process may be limited to a maximum number of iterations for each feature represented in the predictive model. In some implementations, the process may be performed for each feature represented in the predictive model to provide a finalized version of the predictive model that may be stored in the model repository 210.

With particular reference to the example table 400a, a particular feature of a predictive model is considered, in this example, the particular feature having an initial weight *wᵢ* of 0 for the first iteration. In some examples, the predictive model is scored by the model scorer 206 for the case where the feature has an initial weight *wᵢ* of 0. In a first iteration of the process, a first initial momentum *Mᵢ* of 1 is added to the first initial weight *wᵢ* of 0 to provide a test weight *wₜ* of 1. In some examples, the first initial momentum *Mᵢ* may be selected by the model generator 202. In some examples, the first initial momentum *Mᵢ* may be selected as any non-zero scalar value. The predictive model is scored by the model scorer 206 for the case where the feature has a test weight *wₜ* of 1. In this case, the model score improves with respect to the initial model score. That is, the difference between the effective predicted value determined using the adjusted predictive model and the effective actual value, as compared to the difference calculated for the initial predictive model, is reduced. In the example of FIG. 4A, improvement is indicated by a star. Because the test weight *wₜ* of 1 improves the predictive model, the test weight *wₜ* of 1 is kept for use as the initial weight *wᵢ* for the next iteration of the process, and the initial momentum *Mᵢ* is multiplied by a momentum factor of 2 (e.g., binary search is used in this example, so the multiples are provided as 2 and ½) to provide a next momentum *Mₙ* of 2 for application to the initial weight *wᵢ* in the next iteration.

In the next iteration (step 2) of the process, an initial momentum *Mᵢ* of 2 (e.g., the next momentum *Mₙ* provided from step 1) is added to the initial weight *wᵢ* of 1 (e.g., the test weight *wₜ* provided from step 1) to provide a test weight *wₜ* of 3. That is, the initial weight *wᵢ* of 1 is increased by the momentum, which is provided as 2 for the current iteration. The predictive model is scored by the model scorer 206 for the case where the feature has a test weight *wₜ* of 3. In this case, the model score improves with respect to the best model score (the model score calculated in the previous iteration). That is, the difference between the effective predicted value determined using the adjusted predictive model and the effective actual value is again reduced. Because the test weight *wₜ* of 3 improves the predictive model, the test weight *wₜ* of 3 is kept for use as the initial weight *wᵢ* for the next iteration of the process, and the initial momentum *Mᵢ* of 2 is multiplied by the momentum factor of 2 (e.g., binary search is used in this example, so the multiples are provided as 2 and ½) to provide a next momentum *Mₙ* of 4 for application to the initial weight *wᵢ* in the next iteration.

In the next iteration (step 3) of the process, an initial momentum *Mᵢ* i of 4 (e.g., the next momentum *Mₙ* provided from step 2) is added to the initial weight *wᵢ* of 3 (e.g., the test weight *wₜ* provided from step 2) to provide a test weight *wₜ* of 7. That is, the initial weight *wᵢ* of 3 is increased by the momentum, which is provided as 4 for the current iteration. The predictive model is scored by the model scorer 206 for the case where the feature has a test weight *wₜ* of 7. In this case, the model score improves with respect to the best model score (the model score calculated in the previous iteration). That is, the difference between the effective predicted value determined using the adjusted predictive model and the effective actual value is again reduced. Because the test weight *wₜ* of 7 improves the predictive model, the test weight *wₜ* of 7 is kept for use as the initial weight *wᵢ* for the next iteration of the process, and the initial momentum *Mᵢ* of 4 is multiplied by the momentum factor of 2 (e.g., binary search is used in this example, so the multiples are provided as 2 and ½) to provide a next momentum *Mₙ* of 8 for application to the initial weight *wᵢ* in the next iteration.

In the next iteration (step 4) of the process, an initial momentum *Mᵢ* of 8 (e.g., the next momentum *Mₙ* provided from step 3) is added to the initial weight *wᵢ* of 7 (e.g., the test weight *wₜ* provided from step 3) to provide a test weight *wₜ* of 15. That is, the initial weight *wᵢ* of 7 is increased by the momentum, which is provided as 8 for the current iteration. The predictive model is scored by the model scorer 206 for the case where the feature has a test weight *wₜ* of 15. In this case, the model score does not improve with respect to the best model score (the model score calculated in the previous iteration). That is, the difference between the effective predicted value determined using the adjusted predictive model and the effective actual value increases. In the example of FIG. 4A, deterioration is indicated by an X. For example, the test weight *wₜ* is increased too much (e.g., from 7 to 15) in the current iteration, resulting in the predictive model not improving. Because the test weight *wₜ* of 15 does not improve the model, the test weight *wₜ* of 15 is discarded, the initial weight *wᵢ* of 7 is kept for use in the next iteration of the process. In this case, the initial momentum *Mᵢ* of 8 is divided by the momentum factor of 2 (e.g., multiplied by ½, where binary search is used in this example, so the multiples are provided as 2 and ½) to provide a next momentum *Mₙ* of 4 for application to the initial weight *wᵢ* of 7 in the next iteration.

In the next iteration (step 5) of the process, an initial momentum *Mᵢ* of 4 (e.g., the next momentum *Mₙ* provided from step 4) is added to the initial weight *wᵢ* of 7 (e.g., the initial weight *wᵢ* provided from step 4 of the process) to provide a test weight *wₜ* of 11. That is, the initial weight *wᵢ* of 7 is increased by the momentum, which is provided as 4 for the current iteration. The predictive model is scored by the model scorer 206 for the case where the feature has a test weight *wₜ* of 11. In this case, the model score improves with respect to the best model score (the model score calculated in the third iteration (step 3) of the process). That is, the difference between the effective predicted value determined using the adjusted predictive model and the effective actual value, with respect to the difference calculated in the third iteration, is reduced. Because the test weight *wₜ* of 11 improves the predictive model, the test weight *wₜ* of 11 is kept for use as the initial weight *wᵢ* for the next iteration of the process. In this case, the initial momentum *Mᵢ* of 4 is multiplied by the momentum factor of 2 (e.g., binary search is used in this example, so the multiples are provided as 2 and ½) to provide a next momentum *Mₙ* of 8 for application to the initial weight *wᵢ* in the next iteration.

Following the fifth iteration (step 5) of the process, the next example iterations (steps 6-10) of the process may be executed in the same manner as the previous iterations (steps 1-5) to adjust the test weight *wₜ* of the feature according to a next momentum *Mₙ* until the initial momentum *Mᵢ* for a current iteration has returned to 1, but the test weight *wₜ* does not improve the model. This occurs in the eleventh iteration (step 11) of the example of FIG. 4A. For example, referring particularly to step 11, an initial momentum *Mᵢ* of 1 (e.g., the next momentum *Mₙ* provided from step 10) is added to an initial weight *wᵢ* of 12 (e.g., the initial weight *wᵢ* provided from step 10) to provide a test weight *wₜ* of 13. The predictive model is scored by the model scorer 206 for the case where the feature has a test weight *wₜ* of 13. In this case, the model score does not improve with respect to the best model score (the model score calculated in the ninth iteration (step 9) of the process). That is, the difference between the effective predicted value determined using the adjusted predictive model and the effective actual value is greater than the difference calculated in the ninth iteration, such that the model score remains greater than the model score calculated in step 9. Because the test weight *wₜ* of 13 does not improve the predictive model in the case where the initial momentum *Mᵢ* has returned to 1, the test weight *wₜ* of 13 is discarded, the initial weight *wᵢ* of 12 is kept for use as the initial weight *wᵢ* in the next iteration of the process, and a momentum rule is applied to set the next momentum *Mₙ* to 0 for application to the initial weight *wᵢ* in the next iteration.

In the final iteration (step 12) of the depicted example, the process ends because the initial momentum *Mi* (e.g., the next momentum *Mₙ* provided from step 11) is 0. The initial weight *wᵢ* of 12 is kept as the final weight to be applied to the particular feature represented in the predictive model. The corresponding graphical representation illustrates that the process ends, because the initial momentum *Mᵢ* is 0 for the current iteration.

In some implementations, applying a first initial momentum *Mᵢ* of 1 to the first initial weight *wᵢ* may not improve the predictive model. For example, referring particularly to the example table 400b, a predictive model includes a particular feature that has a first initial weight *wᵢ* of 12. The predictive model is scored by the model scorer 206 for the case where the feature has an initial weight *wₜ* of 12. In the first iteration (step 1) of the process, a first initial momentum *Mᵢ* of 1 is added to the first initial weight *wᵢ* of 12 to provide a test weight *wₜ* of 13. The predictive model is scored by the model scorer 206 for the case where the feature has a test weight *wₜ* of 13. In this case, the model score does not improve with respect to the initial model score. That is, the difference between the effective predicted value determined using the adjusted predictive model and the effective actual value is greater than the difference calculated for the initial predictive model, such that the model score is greater than the initial model score. Because the test weight *wₜ* of 13 does not improve the model for the case where the first initial momentum *Mᵢ* is 1, the test weight *wₜ* of 13 is discarded, the initial weight *wᵢ* of 12 is kept for use in the next iteration of the process, and a momentum rule is applied to multiply the initial momentum *Mᵢ* by a momentum factor of -1 to reverse the direction (e.g., from a positive direction to a negative direction) in which the weight is adjusted. A next momentum *Mₙ* of -1 is provided for application to the initial weight *wᵢ* of 12 in the next iteration of the process.

In the next iteration (step 2) of the process, an initial momentum *Mᵢ* of -1 (e.g., the next momentum *Mₙ* provided from step 1) is added to the initial weight *wᵢ* of 12 (e.g., the test weight *wₜ* provided from step 1) to provide a test weight *wₜ* of 11. The predictive model is scored by the model scorer 206 for the case where the feature has a test weight *wₜ* of 11. In this case, the model score improves with respect to the best model score (e.g., the model score is less than the initial model score). Because the test weight *wₜ* of 11 improves the predictive model, the test weight *wₜ* of 11 is kept for use as the initial weight *wᵢ* for the next iteration of the process, and the initial momentum *Mᵢ* of -1 is multiplied by a momentum factor of 2 (e.g., binary search is used in this example, so the multiples are provided as 2 and ½) to provide a next momentum *Mₙ* of -2 for application to the initial weight *wᵢ* in in the next iteration.

In some implementations, if multiplying the first initial momentum *Mᵢ* by a momentum factor of -1 does not improve the model score by a minimum threshold, then the process may be ended and restarted with a different first initial weight *wᵢ*.

Following the second iteration (step 2) of the process, the next example iterations (steps 3-7) of the process may be executed in the same manner as the previous iterations (e.g., recursively) to adjust the test weight *wₜ* of the feature according to a next momentum *Mₙ* until the initial momentum *Mᵢ* has returned to -1, but the test weight *wₜ* does not improve the predictive model. This occurs in step 8 of the example of FIG. 4B. For example, referring particularly to step 8, an initial momentum *Mi* of -1 (e.g., the next momentum *Mₙ* provided from step 7) is added to an initial weight *wᵢ* of 7 (e.g., the initial weight *wᵢ* provided from step 7) to provide a test weight *wₜ* of 6. The predictive model is scored by the model scorer 206 for the case where the feature has a test weight *wₜ* of 6. In this case, the model score does not improve with respect to the best model score (the model score calculated in the fifth iteration (step 5) of the process). That is, the model score remains greater than the model score calculated in step 5. Because the test weight *wₜ* of 6 does not improve the predictive model in the case where the initial momentum *Mᵢ* has returned to -1, the test weight *wₜ* of 6 is discarded, the initial weight *wᵢ* of 7 is kept for use as the initial weight *wᵢ* in the next iteration of the process, and a momentum rule is applied to set the next momentum *Mₙ* to 0 for application to the initial weight *wᵢ* in the next iteration of the process.

In the next iteration (step 9) of the process, the process ends because the initial momentum *Mi* (e.g., the next momentum *Mₙ* provided from step 8) is 0. The initial weight *wᵢ* of 7 is kept as the final weight to be applied to the particular feature represented in the predictive model. The corresponding graphical representation illustrates that the process ends because the initial momentum *Mᵢ* is 0.

In some implementations, adjusting the initial weights *wᵢ* to generate test weights *wₜ*, scoring the predictive model using the test weights *wₜ*, and then keeping the test weights *wₜ*, if the model score improves, provides a modified simulated annealing methodology for improving the predictive model generated by the model generator 202. In some implementations, applying the momentum factor to the initial momentum *Mᵢ* (e.g., multiplying or dividing the initial momentum *Mᵢ* by the momentum factor) to provide the next momentum *Mₙ* provides a search methodology for improving the predictive model generated by the model generator 202. Accordingly, the model generator 202 and the model scorer 206 may perform the process illustrated in the tables 400a, 400b to produce a final version of the predictive model 300a, 300b generated by the model generator 202. In some examples, using such momentum search techniques may significantly reduce the resources (e.g., computational power) and/or time (e.g., from weeks or months to hours) needed to generate a predictive model, as compared to other types of model generation techniques.

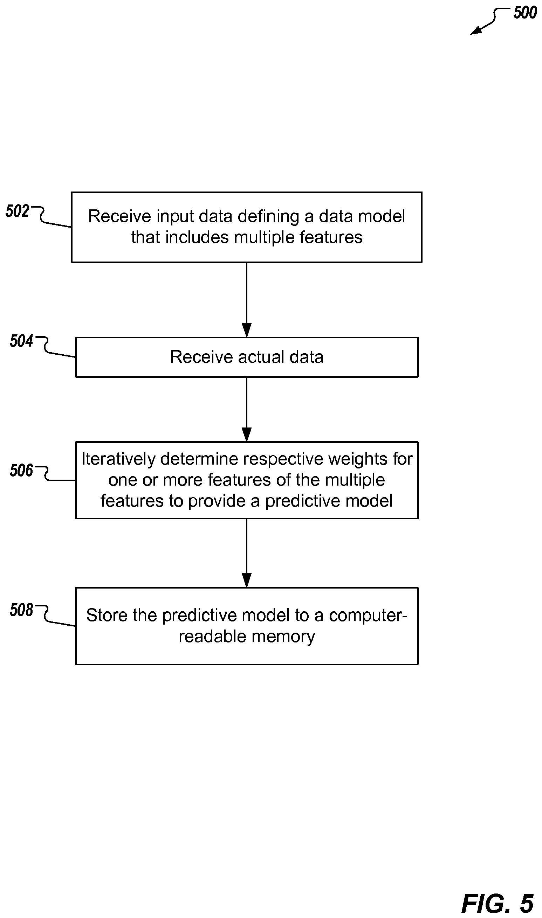

FIG. 5 depicts an example process 500 that can be executed in implementations of the present disclosure. The example process 500 can be implemented, for example, by the example environment 100 of FIG. 1. In some examples, the example process 500 can be provided by one or more computer-executable programs executed using one or more computing devices.

In some implementations, input data defining a data model including multiple features is received (502). For example, the model generator 202 may receive such input from a user to generate a predictive model (e.g., the predictive model 300a, 300b). The predictive model may represent multiple features represented in the actual data stored in the data repository 208. In some example, respective initial weights are provided for one or more features of the predictive model. In some implementations, actual data is received (504). For example, the model scorer 206 may receive the actual data from the data repository 208.

In some implementations, respective weights for one or more features of the multiple features are iteratively determined to provide a predictive model (506). For example, the model generator 202 may iteratively adjust weights corresponding to the features represented in the predictive model based on the model score iteratively generated by the model scorer 206. In some examples, the respective weights are iteratively determined such that, for each feature, a current weight for the feature is iteratively adjusted by a momentum until the momentum equals zero, where the momentum is iteratively adjusted by a multiple based on whether a model score improves, and where the model score is calculated using the actual data. In some examples, the respective weights are further iteratively determined such that, for each feature, the current weight is assigned as a final weight for the feature when the momentum equals zero. For example, the model generator 202 may iteratively adjust the initial weight *wᵢ* for the respective feature by adding an initial momentum *Mᵢ* to the initial weight *wᵢ* until the best model score calculated by the model scorer 206 does not improve. In some examples, the model generator 202 may implement a momentum rule that sets the next momentum *Mₙ* equal to zero once the test weight *wₜ* no longer improves the predictive model in the case where the initial momentum *Mᵢ* has returned to a value of 1 or -1. In some examples, the model generator 202 may iteratively multiply or divide the initial momentum *Mᵢ* by a momentum factor (e.g., 2) to obtain a next momentum *Mₙ*.

In some implementations, the predictive model is stored to a computer-readable memory (508). For example, the model scorer 206 may provide the predictive model to the model repository 210 once finalized weights have been iteratively determined for each feature represented in the model (e.g., once a finalized version of the predictive model has been determined).

In some implementations, a tree-based model 300b may be processed according to a methodology illustrated by the example tables of 400a, 400b by selecting (e.g., in some cases, randomly selecting) a node for which to adjust feature weights. The methodology may then be implemented to adjust the weight of each feature weight included in the node. In some examples, the methodology may be implemented for each node in the tree. In some implementations, processing of the tree-based model 300b may include one or more of adding nodes, removing nodes, adding features to a node, removing features from a node, changing an operation of a node, and changing a comparative value of a node. If such a change improves the model (e.g., based on the methodology illustrated by the example tables of 400a, 400b to adjust a feature weight), then the change is maintained, whereas if the change does not improve the model, then the change is discarded.

In some implementations, only records that are potentially affected by an adjustment to the predictive model are re-scored. For example, upon making a change to a node of the tree-based model 300b, the change may be scored using what-if scoring. In some examples, what-if scoring can allow the algorithm to determine the impact of potential changes without having to actually change the model 300b. In some examples, what-if scoring re-calculates only parts of the model 300b that have actually changed. In such cases, intermediate values may be cached (e.g., saved). In some implementations, what-if scoring re-calculates only the scores for records that the change affects. In some examples, such scoring can significantly improve performance.

In some implementations, the model 300b may be scored using feature-record mapping. For example, the model 300b may reference a list of records that each include a particular feature. In some examples, whenever a weight for the particular feature is changed, only the records that include the particular feature need to be re-scored. Such scoring may improve performance (e.g., in some cases, by a factor of 100), especially in medical applications, where many diagnoses have relatively low incidences.

In some implementations, a comparison of finalized weights included in the predictive model may identify which features (e.g., medical parameters or other patient characteristics) are likely to contribute to the target parameter (e.g., an unplanned hospitalization) represented in the actual data. In some implementations, and within the example context described above, one or more service providers (e.g., medical practitioners and insurance providers) may use a patient hospitalization risk as predicted by the predictive model to determine how to formulate or modify services provided to the patient. In some examples, with reference to a threshold risk, a service provider can use the predictive model to identify patients that are susceptible to an unplanned hospitalization. For example, patients that have a predicted hospitalization risk greater than a threshold risk may be classified as high-risk patients, whereas patients that have a predicted hospitalization risk less than or equal to the threshold risk may be classified as low-risk patients.

In some implementations, a hospitalization risk calculated by the predictive model may be provided to one or more medical records systems (e.g., integrated electronic medical records (EMR) systems). In some examples, a medical practitioner may access a patient's hospitalization risk from the one or more medical records systems and consider the hospitalization risk as a factor in generating a prognosis or developing a treatment plan for the patient. For example, the medical practitioner may choose to schedule one or more follow-up medical appointments for the patient based on a high hospitalization risk (e.g., with reference to a threshold risk). In some implementations, a high hospitalization risk may trigger a notification indicating the risk for the particular patient. In some examples, the notification may be provided to the medical practitioner via the one or more medical records systems. In some examples, a notification indicating a high risk may be provided directly to the patient (e.g., via an e-mail, text message, or voice message).

Implementations and all of the functional operations described in this specification may be realized in digital electronic circuitry, or in computer software, firmware, or hardware, including the structures disclosed in this specification and their structural equivalents, or in combinations of one or more of them. Implementations may be realized as one or more computer program products, i.e., one or more modules of computer program instructions encoded on a computer readable medium for execution by, or to control the operation of, data processing apparatus. The computer readable medium may be a machine-readable storage device, a machine-readable storage substrate, a memory device, a composition of matter effecting a machine-readable propagated signal, or a combination of one or more of them. The term "computing system" encompasses all apparatus, devices, and machines for processing data, including by way of example a programmable processor, a computer, or multiple processors or computers. The apparatus may include, in addition to hardware, code that creates an execution environment for the computer program in question, *e.g.,* code that constitutes processor firmware, a protocol stack, a database management system, an operating system, or a combination of one or more of them. A propagated signal is an artificially generated signal, *e.g.,* a machine-generated electrical, optical, or electromagnetic signal that is generated to encode information for transmission to suitable receiver apparatus.

A computer program (also known as a program, software, software application, script, or code) may be written in any appropriate form of programming language, including compiled or interpreted languages, and it may be deployed in any appropriate form, including as a stand alone program or as a module, component, subroutine, or other unit suitable for use in a computing environment. A computer program does not necessarily correspond to a file in a file system. A program may be stored in a portion of a file that holds other programs or data (*e.g*., one or more scripts stored in a markup language document), in a single file dedicated to the program in question, or in multiple coordinated files (*e.g*., files that store one or more modules, sub programs, or portions of code). A computer program may be deployed to be executed on one computer or on multiple computers that are located at one site or distributed across multiple sites and interconnected by a communication network.

The processes and logic flows described in this specification may be performed by one or more programmable processors executing one or more computer programs to perform functions by operating on input data and generating output. The processes and logic flows may also be performed by, and apparatus may also be implemented as, special purpose logic circuitry, *e.g*., an FPGA (field programmable gate array) or an ASIC (application specific integrated circuit).

Processors suitable for the execution of a computer program include, by way of example, both general and special purpose microprocessors, and any one or more processors of any appropriate kind of digital computer. Generally, a processor will receive instructions and data from a read only memory or a random access memory or both. Elements of a computer can include a processor for performing instructions and one or more memory devices for storing instructions and data. Generally, a computer will also include, or be operatively coupled to receive data from or transfer data to, or both, one or more mass storage devices for storing data, *e.g*., magnetic, magneto optical disks, or optical disks. However, a computer need not have such devices. Moreover, a computer may be embedded in another device, *e.g.,* a mobile telephone, a personal digital assistant (PDA), a mobile audio player, a Global Positioning System (GPS) receiver, to name just a few. Computer readable media suitable for storing computer program instructions and data include all forms of non-volatile memory, media and memory devices, including by way of example semiconductor memory devices, *e.g.,* EPROM, EEPROM, and flash memory devices; magnetic disks, *e.g.,* internal hard disks or removable disks; magneto optical disks; and CD ROM and DVD-ROM disks. The processor and the memory may be supplemented by, or incorporated in, special purpose logic circuitry.

To provide for interaction with a user, implementations may be realized on a computer having a display device, *e.g.,* a CRT (cathode ray tube) or LCD (liquid crystal display) monitor, for displaying information to the user and a keyboard and a pointing device, *e.g.,* a mouse or a trackball, by which the user may provide input to the computer. Other kinds of devices may be used to provide for interaction with a user as well; for example, feedback provided to the user may be any appropriate form of sensory feedback, *e.g*., visual feedback, auditory feedback, or tactile feedback; and input from the user may be received in any appropriate form, including acoustic, speech, or tactile input.

Implementations may be realized in a computing system that includes a back end component, *e.g.,* as a data server, or that includes a middleware component, *e.g.,* an application server, or that includes a front end component, *e.g*., a client computer having a graphical user interface or a Web browser through which a user may interact with an implementation, or any appropriate combination of one or more such back end, middleware, or front end components. The components of the system may be interconnected by any appropriate form or medium of digital data communication, *e.g*., a communication network. Examples of communication networks include a local area network ("LAN") and a wide area network ("WAN"), *e.g.,* the Internet.

The computing system may include clients and servers. A client and server are generally remote from each other and typically interact through a communication network. The relationship of client and server arises by virtue of computer programs running on the respective computers and having a client-server relationship to each other.

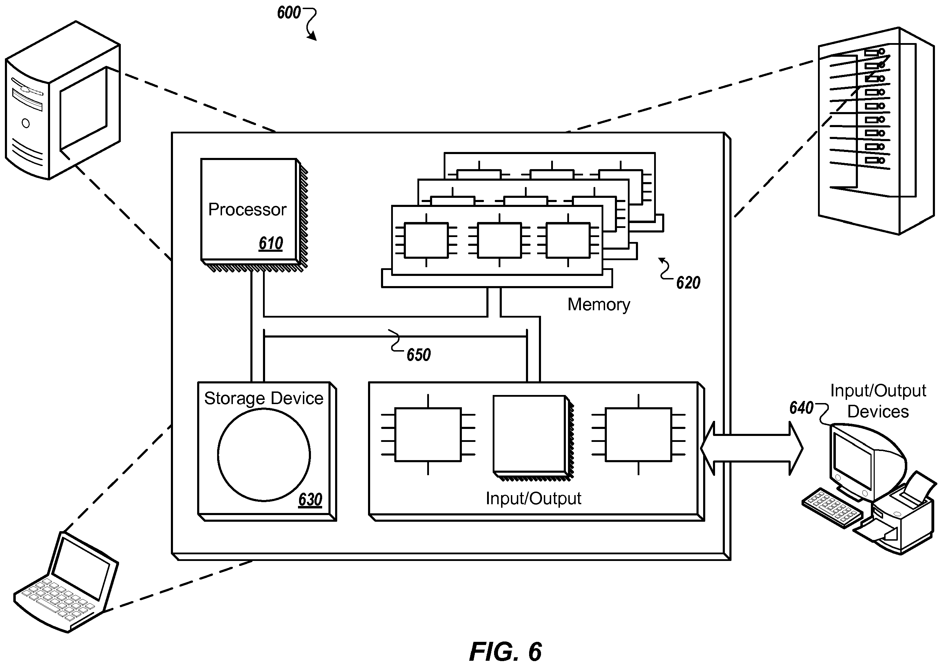

FIG. 6 depicts an example computing system 600 that can execute implementations of the present disclosure. The system 600 can be used for the operations described in association with any of the computer-implement methods described previously, according to one implementation. The system 600 includes a processor 610, a memory 620, a storage device 630, and an input/output device 640. Each of the components 610, 620, 630, and 640 are interconnected using a system bus 650. The processor 610 is capable of processing instructions for execution within the system 600. In one implementation, the processor 610 is a single-threaded processor. In another implementation, the processor 610 is a multi-threaded processor. The processor 610 is capable of processing instructions stored in the memory 620 or on the storage device 630 to display graphical information for a user interface on the input/output device 640.

The memory 620 stores information within the system 600. In one implementation, the memory 620 is a computer-readable medium. In one implementation, the memory 620 is a volatile memory unit. In another implementation, the memory 620 is a non-volatile memory unit.

The storage device 630 is capable of providing mass storage for the system 600. In one implementation, the storage device 630 is a computer-readable medium. In various different implementations, the storage device 630 may be a floppy disk device, a hard disk device, an optical disk device, or a tape device.

The input/output device 640 provides input/output operations for the system 600. In one implementation, the input/output device 640 includes a keyboard and/or pointing device. In another implementation, the input/output device 640 includes a display unit for displaying graphical user interfaces.

While this specification contains many specifics, these should not be construed as limitations on the scope of the disclosure or of what may be claimed, but rather as descriptions of features specific to particular implementations. Certain features that are described in this specification in the context of separate implementations may also be implemented in combination in a single implementation. Conversely, various features that are described in the context of a single implementation may also be implemented in multiple implementations separately or in any suitable sub-combination. Moreover, although features may be described above as acting in certain combinations and even initially claimed as such, one or more features from a claimed combination may in some cases be excised from the combination, and the claimed combination may be directed to a sub-combination or variation of a sub-combination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the implementations described above should not be understood as requiring such separation in all implementations, and it should be understood that the described program components and systems may generally be integrated together in a single software product or packaged into multiple software products.

A number of implementations have been described. Nevertheless, it will be understood that various modifications may be made without departing from the scope of the disclosure. For example, various forms of the flows shown above may be used, with steps re-ordered, added, or removed. Accordingly, other implementations are within the scope of the following claims.

## Claims

1. A method for predicting a hospitalization risk associated with a patient, the method being executed by one or more processors and comprising:
receiving, by the one or more processors, input data defining a tree based model (300b), wherein the tree based model (300b):
comprises a root node, intermediate nodes and terminal nodes, wherein the root node corresponds to a hospitalization risk value and the intermediate nodes and terminal nodes correspond to a plurality of features, the plurality of features comprising biological parameters, medical conditions, patient characteristics, or medical history parameters, and wherein each node:
is associated with a multiplier;
accounts for values for multiple features of the plurality of features that are respectively associated with multiple weights,
accounts for any child nodes directly or indirectly associated with the node, and
wherein the tree based model (300b) is traversed from a terminal node to the root node to calculate a predicted hospitalization risk value by multiplying a result of each node by its associated multiplier;
weighting, by the one or more processors, the tree based model (300b) iteratively for each feature of the plurality of features, using hospitalization data comprising, for each of a plurality of patients within a population that were previously hospitalized:
a hospitalization risk value, and
a value for each of the plurality of features,
the weighting comprising, for each feature of the plurality of features:
iteratively adjusting a current weight (404a, 404b) corresponding to the feature by adding a momentum (406a, 406b) to the current weight (404a, 404b), the momentum being iteratively multiplied or divided by a momentum factor based on whether a model score improves or not and until the momentum equals zero, the model score being calculated based on the hospitalization data, wherein the model score comprises a difference between a predicted value determined using the tree based model and a value determined from the hospitalization data, and the model score improves when the difference for a current iteration decreases as compared to a difference corresponding to the previously calculated best model score; and
calculating, by the one or more processors, a hospitalization risk score for the patient using the weighted tree based model (300b).

2. The method of claim 1, wherein iteratively multiplying the momentum by a momentum factor based on whether a model score improves or not comprises performing binary search.

3. The method of claim 1, wherein iteratively adjusting a current weight (404a, 404b) corresponding to the feature by adding a momentum to the current weight comprises using previously determined weights for each feature of the plurality of features as initial weights for each feature of the plurality of features in a current iteration.

4. The method of claim 1, wherein the tree based model (300b) further comprises respective initial weights assigned to one or more features of the plurality of features, for example, wherein one or more of the respective initial weights equals 1 or one or more of the respective initial weights equals 0.

5. The method of claim 1, wherein the plurality of features represent one or more of medical parameters and demographic parameters, for example, wherein the medical parameters comprise one or more of DSM codes, ICD-9 codes, ICD-10 codes, SNOMED codes, LOINC codes, RxNORM codes, CPT codes, and non-codified parameters.

6. The method of claim 1, further comprising generating the tree based model (300b) based on the input data.

7. The method of claim 1, wherein the model score is compared to a previously calculated best model score

8. The method of claim 1, wherein the current weight is adjusted in a positive direction.

9. The method of claim 1, wherein a momentum rule is applied to set the momentum to -1 if, for an initial momentum of 1, the model score does not improve by a threshold value, for example, wherein the current weight is adjusted in a negative direction.

10. The method of claim 1, wherein the current weight for the feature is adjusted by the momentum until a maximum number of iterations has been reached for adjusting the current weight.

11. The method of claim 1, wherein a momentum rule is applied to set the momentum to zero once the momentum returns to a value of 1 or -1 for the case where the model score no longer improves.

12. The method of claim 1, wherein iterations for determining the respective weights are limited to a maximum number of iterations for each feature of the plurality of features.

13. The method of claim 1, wherein the hospitalization risk score is either provided to a medical records system or used by a service provider to formulate one or more services for the patient, optionally wherein medical practitioners use the hospitalization risk score to generate one or more of a prognosis and a treatment plan for the patient, or a payer uses the hospitalization risk to allocate medical resources within a treatment facility.

14. A non-transitory computer-readable storage medium coupled to one or more processors and having instructions stored thereon which, when executed by the one or more processors, cause the one or more processors to perform operations for determining unplanned hospitalization risk for a patient, the operations comprising the method of any one of claims 1 to 13.

15. A system, comprising:
one or more processors; and
a computer-readable storage device coupled to the one or more processors and having instructions stored thereon which, when executed by the one or more processors, cause the one or more processors to perform operations for determining unplanned hospitalization risk for a patient, the operations comprising the method of any one of claims 1 to 13.

## Patentansprüche

1. Verfahren zum Vorhersagen eines Krankenhausaufenthaltsrisikos im Zusammenhang mit einem Patienten, wobei das Verfahren von einem oder mehreren Prozessoren ausgeführt wird und umfasst:
Empfangen in dem einen oder den mehreren Prozessoren von Eingangsdaten, die ein baumstrukturbasiertes Modell (300b) definieren, wobei das baumstrukturbasierte Modell (300b) :
einen Wurzelknoten, zwischengeschaltete Knoten und Endknoten umfasst, wobei der Wurzelknoten einem Krankenhausaufenthaltsrisikowert entspricht und die zwischengeschalteten Knoten und die Endknoten einer Vielzahl von Merkmalen entsprechen, wobei die Vielzahl von Merkmalen biologische Parameter, medizinische Bedingungen, Patienteneigenschaften oder Parameter der Krankengeschichte umfassen, und wobei jeder Knoten:
einen zugeordneten Faktor aufweist,
Werte für mehrere Merkmale aus der Vielzahl von Merkmalen berücksichtigt, denen jeweils mehrere Gewichtungen zugeordnet sind,
jeden untergeordneten Knoten berücksichtigt, der dem Knoten direkt oder indirekt zugeordnet ist, und
wobei das baumstrukturbasierte Modell (300b) von einem Endknoten zu dem Wurzelknoten durchlaufen wird, um einen vorhergesagten Krankenhausaufenthaltsrisikowert zu berechnen, indem ein Ergebnis von jedem Knoten mit seinem zugeordneten Faktor multipliziert wird;
iteratives Gewichten mithilfe des einen oder der mehreren Prozessoren des baumstrukturbasierten Modells (300b) für jedes Merkmal aus der Vielzahl von Merkmalen mithilfe von Krankenhausaufenthaltsdaten, die für jeden aus einer Vielzahl von Patienten innerhalb einer Bevölkerungsgruppe, die zuvor einen Krankenhausaufenthalt absolviert haben, umfassen:
einen Krankenhausaufenthaltsrisikowert, und
einen Wert für jeden aus der Vielzahl von Merkmalen, wobei das Gewichten für jedes Merkmal aus der Vielzahl von Merkmalen umfasst:
iteratives Einstellen einer aktuellen Gewichtung (404a, 404b), die dem Merkmal entspricht, indem ein dynamischer Wert (406a, 406b) zu der Gewichtung (404a, 404b) addiert wird, wobei der dynamische Wert, aufgrund dessen, ob sich eine Modellpunktzahl verbessert oder nicht, mit einem Faktor des dynamischen Werts iterativ multipliziert oder durch diesen iterativ dividiert wird, bis der dynamische Wert gleich Null ist, wobei die Modellpunktzahl aufgrund der Krankenhausaufenthaltsdaten berechnet wird, wobei die Modellpunktzahl eine Differenz zwischen einem vorhergesagten Wert, der mithilfe des baumstrukturbasierten Modells ermittelt wird, und einem Wert umfasst, der aus den Krankenhausaufenthaltsdaten ermittelt wird, und wobei sich die Modellpunktzahl verbessert, wenn die Differenz für eine aktuelle Iteration im Vergleich zu einer Differenz abnimmt, die der zuvor berechneten besten Modellpunktzahl entspricht, und
Berechnen durch den einen oder die mehreren Prozessoren einer Krankenhausaufenthaltsrisikopunktzahl für den Patienten mithilfe des gewichteten baumstrukturbasierten Modells (300b).

2. Verfahren nach Anspruch 1, wobei das iterative Multiplizieren des dynamischen Werts mit einem Faktor des dynamischen Werts, aufgrund dessen, ob sich eine Modellpunktzahl verbessert oder nicht, ein Ausführen einer binären Suche umfasst.

3. Verfahren nach Anspruch 1, wobei das iterative Einstellen einer aktuellen Gewichtung (404a, 404b), die dem Merkmal entspricht, indem ein dynamischer Wert zu der aktuellen Gewichtung addiert wird, ein Verwenden zuvor ermittelter Gewichtungen für jedes Merkmal aus der Vielzahl von Merkmalen als anfängliche Gewichtungen für jedes Merkmal aus der Vielzahl von Merkmalen in einer aktuellen Iteration umfasst.

4. Verfahren nach Anspruch 1, wobei das baumstrukturbasierte Modell (300b) außerdem zum Beispiel jeweilige anfängliche Gewichtungen umfasst, die einem oder mehreren Merkmalen aus der Vielzahl von Merkmalen zugewiesen wurden, wobei eine oder mehrere der jeweiligen anfänglichen Gewichtungen gleich 1 sind oder eine oder mehrere der jeweiligen anfänglichen Gewichtungen gleich 0 sind.

5. Verfahren nach Anspruch 1, wobei die Vielzahl von Merkmalen zum Beispiel einen oder mehrere von medizinische Parametern oder demografischen Parametern darstellt, wobei die medizinischen Parameter einen oder mehrere von DSM-Codes, ICD-9-Codes, ICD-10-Codes, SNOMED-Codes, LOINC-Codes, RxNORM-Codes, CPT-Codes und nichtcodifizierte Parameter umfassen.

6. Verfahren nach Anspruch 1, das außerdem ein Erzeugen des baumstrukturbasierten Modells (300b) aufgrund der Eingangsdaten umfasst.

7. Verfahren nach Anspruch 1, wobei die Modellpunktzahl mit einer zuvor berechneten besten Modellpunktzahl verglichen wird.

8. Verfahren nach Anspruch 1, wobei die aktuelle Gewichtung in eine positive Richtung justiert wird.

9. Verfahren nach Anspruch 1, wobei eine Regel für dynamische Werte angewandt wird, um den dynamischen Wert auf -1 zu setzen, wenn sich die Modellpunktzahl für einen anfänglichen dynamischen Wert von 1 zum Beispiel mithilfe eines Schwellenwerts nicht verbessert, wobei die aktuelle Gewichtung in eine negative Richtung justiert wird.

10. Verfahren nach Anspruch 1, wobei die aktuelle Gewichtung für das Merkmal mithilfe des dynamischen Werts justiert wird, bis eine maximale Anzahl von Iterationen zum Justieren der aktuellen Gewichtung erreicht wird.

11. Verfahren nach Anspruch 1, wobei eine Regel für dynamische Werte angewandt wird, um den dynamischen Wert auf Null zu setzen, sobald der dynamische Wert für den Fall, bei dem die Modellpunktzahl nicht mehr verbessert wird, auf einen Wert von 1 oder -1 zurückkehrt.

12. Verfahren nach Anspruch 1, wobei die Iterationen zum Ermitteln der jeweiligen Gewichtungen auf eine maximale Anzahl von Iterationen für jedes Merkmal aus der Vielzahl von Merkmalen begrenzt sind.

13. Verfahren nach Anspruch 1, wobei die Krankenhausaufenthaltsrisikopunktzahl entweder einem Krankenberichtssystem bereitgestellt wird oder von einem Dienstanbieter verwendet wird, um eine oder mehrere Dienstleistungen für den Patienten zu formulieren, wobei optional Ärzte die Krankenhausaufenthaltsrisikopunktzahl verwenden, um eine oder mehrere einer Prognose und eines Behandlungsplans für den Patienten zu erzeugen, oder ein Zahlungsträger das Krankenhausaufenthaltsrisiko verwendet, um einer Behandlungseinrichtung medizinische Ressourcen zuzuweisen.

14. Nichtflüchtiges computerlesbares Speichermedium, das mit einem oder mehreren Prozessoren verbunden ist und Befehle aufweist, die darin gespeichert sind und die, wenn sie von dem einen oder den mehreren Prozessoren ausgeführt werden, den einen oder die mehreren Prozessoren veranlassen, Funktionen zum Ermitteln eines ungeplanten Krankenhausaufenthaltsrisikos für einen Patienten auszuführen, wobei die Funktionen das Verfahren nach einem der Ansprüche 1 bis 13 umfassen.

15. System, umfassend:
einen oder mehrere Prozessoren; und
eine computerlesbare Speichervorrichtung, die mit dem einen oder den mehreren Prozessoren verbunden ist und Befehle aufweist, die darin gespeichert sind und die, wenn sie von dem einen oder den mehreren Prozessoren ausgeführt werden, den einen oder die mehreren Prozessoren veranlassen, Funktionen zum Ermitteln eines ungeplanten Krankenhausaufenthaltsrisikos für einen Patienten auszuführen, wobei die Funktionen das Verfahren nach einem der Ansprüche 1 bis 13 umfassen.

## Revendications

1. Procédé de prédiction d'un risque d'hospitalisation associé à un patient, le procédé étant exécuté par un ou plusieurs processeurs et comprenant les étapes suivantes :
recevoir, par les un ou plusieurs processeurs, des données d'entrée définissant un modèle arborescent (300b), où le modèle arborescent (300b) :
comprend un noeud racine, des noeuds intermédiaires et des noeuds terminaux, où le noeud racine correspond à une valeur de risque d'hospitalisation et les noeuds intermédiaires et les noeuds terminaux correspondent à une pluralité de caractéristiques, la pluralité de caractéristiques comprenant des paramètres biologiques, des états de santé, des caractéristiques du patient ou des paramètres d'historique médical, et où chaque noeud :
est associé à un multiplicateur ;
prend en compte les valeurs de plusieurs caractéristiques de la pluralité de caractéristiques qui sont respectivement associées à des pondérations multiples,
prend en compte tous les noeuds enfants directement ou indirectement associés au noeud, et
où le modèle arborescent (300b) est traversé d'un noeud terminal au noeud racine pour calculer une valeur de risque d'hospitalisation prédite en multipliant un résultat de chaque noeud par son multiplicateur associé ;
pondérer, par les un ou plusieurs processeurs, le modèle arborescent (300b) de façon itérative pour chaque caractéristique de la pluralité de caractéristiques, en utilisant des données d'hospitalisation comprenant, pour chacun d'une pluralité de patients d'une population qui ont été précédemment hospitalisés :
une valeur de risque d'hospitalisation, et
une valeur pour chacune de la pluralité de caractéristiques,
la pondération comprenant, pour chaque caractéristique de la pluralité de caractéristiques :
l'ajustement itératif d'une pondération courante (404a, 404b) correspondant à la caractéristique en ajoutant un moment (406a, 406b) à la pondération courante (404a, 404b), le moment étant multiplié ou divisé par un facteur de moment sur la base du fait qu'un score de modèle s'améliore ou non et jusqu'à ce que le moment soit égal à zéro, le score de modèle étant calculé sur la base des données d'hospitalisation, où le score de modèle comprend une différence entre une valeur prédite déterminée en utilisant le modèle arborescent et une valeur déterminée à partir des données d'hospitalisation, et le score de modèle s'améliore lorsque la différence pour une itération courante diminue par rapport à une différence correspondant au meilleur score de modèle calculé précédemment ; et
calculer, par les un ou plusieurs processeurs, un score de risque d'hospitalisation pour le patient au moyen du modèle arborescent pondéré (300b).

2. Procédé selon la revendication 1, dans lequel la multiplication itérative du moment par un facteur de moment sur la base du fait qu'un score de modèle s'améliore ou non comprend de procéder à une recherche binaire.

3. Procédé selon la revendication 1, dans lequel l'ajustement itératif d'une pondération courante (404a, 404b) correspondant à la caractéristique par addition d'un moment à la pondération courante comprend d'utiliser les pondérations préalablement déterminées pour chaque caractéristique de la pluralité de caractéristiques comme pondérations initiales pour chaque caractéristique de la pluralité de caractéristiques dans une itération courante.

4. Procédé selon la revendication 1, dans lequel le modèle arborescent (300b) comprend en outre des pondérations initiales respectives affectées à une ou plusieurs caractéristiques de la pluralité de caractéristiques, par exemple, où un ou plusieurs des pondérations initiales respectives sont égales à 1 ou une ou plusieurs des pondérations initiales respectives sont égales à 0.

5. Procédé selon la revendication 1, dans lequel la pluralité de caractéristiques représentent un ou plusieurs paramètres médicaux et paramètres démographiques, par exemple, où les paramètres médicaux comprennent un ou plusieurs des codes DSM, des codes ICD-9, des codes ICD-10, des codes SNOMED, des codes LOINC, des codes RxNORM, des codes CPT et des paramètres non-codifiés.

6. Procédé selon la revendication 1, comprenant en outre de générer le modèle arborescent (300b) sur la base des données d'entrée.

7. Procédé selon la revendication 1, dans lequel le score de modèle est comparé au meilleur score de modèle calculé précédemment.

8. Procédé selon la revendication 1, dans lequel la pondération courante est ajustée dans une direction positive.

9. Procédé selon la revendication 1, dans lequel une règle de moment est appliquée pour définir le moment à -1 si, pour un moment initial de 1, le score de modèle ne s'améliore pas d'une valeur seuil, par exemple, où la pondération courante est ajustée dans une direction négative.

10. Procédé selon la revendication 1, dans lequel la pondération courante de la caractéristique est ajustée par le moment jusqu'à ce qu'un nombre maximum d'itérations ait été atteint pour ajuster la pondération courante.

11. Procédé selon la revendication 1, dans lequel une règle de moment est appliquée pour définir le moment à zéro lorsque le moment revient à une valeur de 1 ou de -1 dans le cas où le score de modèle ne s'améliore plus.

12. Procédé selon la revendication 1, dans lequel les itérations pour déterminer les pondérations respectives sont limitées à un nombre maximum d'itérations pour chaque caractéristique de la pluralité de caractéristiques.

13. Procédé selon la revendication 1, dans lequel le score de risque d'hospitalisation est soit fourni à un système de dossiers médicaux, soit utilisé par un prestataire de services pour formuler un ou plusieurs services pour le patient, optionnellement où les médecins utilisent le score de risque d'hospitalisation pour générer un ou plusieurs avis parmi un pronostic et un plan de traitement pour le patient, ou un payeur utilise le risque d'hospitalisation pour allouer les frais médicaux dans un établissement de soins.

14. Support de stockage non transitoire lisible par ordinateur couplé à un ou plusieurs processeurs et comportant des instructions stockées sur celui-ci qui, lorsqu'elles sont exécutées par les un ou plusieurs processeurs, amènent les un ou plusieurs processeurs à exécuter des opérations pour déterminer le risque d'hospitalisation non planifiée pour un patient, les opérations comprenant le procédé de l'une quelconque des revendications 1 à 13.

15. Système, comprenant :
un ou plusieurs processeurs ; et
un dispositif de stockage lisible par ordinateur couplé aux un ou plusieurs processeurs et sur lequel sont stockées des instructions qui, lorsqu'elles sont exécutées par les un ou plusieurs processeurs, amènent les un ou plusieurs processeurs à exécuter des opérations pour déterminer le risque d'hospitalisation non planifiée d'un patient, les opérations comprenant le procédé de l'une quelconque des revendications 1 à 13.
